(19) Europäisches Patentamt
European Patent Office
Office européen des brevets

(11) Numéro de publication: **0 043 303**
**B1**

(12) **FASCICULE DE BREVET EUROPEEN**

(45) Date de publication du fascicule du brevet: **21.09.83**

(51) Int. Cl.³: **C 07 C 25/02**, C 07 C 17/00

(21) Numéro de dépôt: **81400952.8**

(22) Date de dépôt: **16.06.81**

(54) **Procédé d'isomérisation et/ou de réarrangement de polychlorobenzènes.**

(30) Priorité: **01.07.80 FR 8014607**
**12.05.81 FR 8109392**

(43) Date de publication de la demande:
*06.01.82 Bulletin 82/1*

(45) Mention de la délivrance du brevet:
**21.09.83 Bulletin 83/38**

(84) Etats contractants désignés:
*AT BE CH DE FR GB IT LI LU NL SE*

(56) Documents cités:
FR-A-1 061 506
US-A-2 866 829

JOURNAL OF THE AMERICAN CHEMICAL SOCIETY, vol. 93, 10 mars 1971, EASTON (US) J.F. BUNNETT et al.: "Isomerization and Disproportionation of Trihalobenzenes Catalyzed by Potassium Analide in Liquid Ammonia", pages 1183-1190

(73) Titulaire: **RHONE-POULENC SPECIALITES CHIMIQUES, "Les Miroirs" 18, Avenue d'Alsace, F-92400 Courbevoie (FR)**

(72) Inventeur: *Soula, Gérard, 33, rue Nungesser, F-69330 - Meyzieu (FR)*

(74) Mandataire: **Cazes, Jean-Marie et al, RHONE-POULENC RECHERCHES Service Brevets Chimie et Polymères 25, quai Paul Doumer, F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 043 303 B1

# Procédé d'isomérisation et/ou réarrangement de polychlorobenzènes

La présente invention a pour objet un procédé d'isomérisation et/ou de réarrangement de polychlorobenzènes. Elle concerne plus particulièrement un procédé de préparation de polychlorobenzènes par réarrangement et/ou isomérisation de polychlorobenzènes.

Ce type de réaction est connue dans le cadre de polybromobenzènes. C'est ainsi que J.F. Bunnett et Charles E. Moyer Jr décrivent dans «Journal of the American Chemical Society» 93:5 du 10 mars 1971 pages 1183–1205 la réaction de l'amidure de sodium dans l'ammoniac liquide sur le tribromo-1,2,4 benzène. Ce dernier se réarrange en tribromo-1,3,5 benzène. On obtient également une petite quantité de dérivés di- et tetrabromés. De la même façon, cette publication décrit l'obtention, à partir du iodo-1 dibromo-2,4 benzène, du mélange contenant l'isomère 1,3,5 ainsi que le tribromo-1,2,4 benzène, le tribromo-1,3,5 benzène et le bromo-1 diodo-3,5 benzène. Cette même publication décrit encore la réaction de $NaNH_2$ dans l'ammoniac liquide avec le bromo-1 dichloro-2,4 benzène, le iodo-1 bromo-4 chlorobenzène et le iodo-1 tribromo-2,4,6 benzène.

Les auteurs précités ont tenté de faire réagir, dans les mêmes conditions, des polychlorobenzènes (plus particulièrement, le trichloro-1,2,4 benzène). Cette tentative est [illegible] restée infructueuse (page 1185 colonne de droite).

Le procédé selon l'invention atteint cet objectif et réalise donc une réaction qui n'avait jamais pu être mise en œuvre dans l'art antérieur.

La présente invention a donc pour objet un procédé d'isomérisation et/ou de réarrangement de polychlorobenzènes caractérisé en ce que l'on met en réaction d'une part:

- au moins un tétrachlorobenzène éventuellement en mélange avec au moins un trichlorobenzène et/ou le pentachlorobenzène et/ou l'hexachlorobenzène.
- ou le pentachlorobenzène en mélange avec au moins un trichlorobenzène et/ou l'hexachlorobenzène.
- ou l'hexachlorobenzène en mélange avec au moins un trichlorobenzène et/ou un dichlorobenzène et, d'autre part, au moins un amidure alcalin et/ou au moins un alcoolate alcalin en présence d'au moins un composé complexant le cation de l'amidure et/ou l'alcoolate.

Selon un premier mode de mise en œuvre particulier de l'invention, le composé complexant le cation de l'amidure alcalin et/ou l'alcoolate est un agent séquestrant de formule:

$$N\text{-}[CHR_1\text{-}CHR_2\text{-}O\text{-}(CHR_3\text{-}CHR_4\text{-}O)_n\text{-}R_5]_3 \qquad (I)$$

dans laquelle n est un nombre entier supérieur ou égal à 0 et inférieur ou égal à 10 ($0 \leq n \leq 10$), $R_1$, $R_2$, $R_3$ et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $-C_mH_{2m}-\varnothing$ ou $C_mH_{2m+1}-\varnothing-$, où m est compris entre 1 et 12 ($1 \leq m \leq 12$).

Selon un deuxième mode de mise en œuvre particulier de l'invention, le composé complexant est un polyéther macrocyclique ayant de 15 à 30 atomes dans le cycle et constitué de 4 à 10 unités -O-X dans lesquelles X est soit $-CHR_6-CHR_7-$ soit $-CHR_6-CHR_8-CR_9R_7-$, $R_6$, $R_7$, $R_8$ et $R_9$ identiques ou différents étant un atome d'hydrogène ou un radical alkyle ayant de 1 à 4 atomes de carbone, un des X pouvant être $-CHR_6-CHR_8-CR_9R_7-$ quand les unités -O-X comprennent le groupement -O-CHR -CHR [illegible] $_6$ [illegible] $R_7$-.

Selon un troisième mode de mise en œuvre particulier de l'invention, le composé complexant est un composé macrocyclique ou bicyclique de formule générale IIa ou IIb

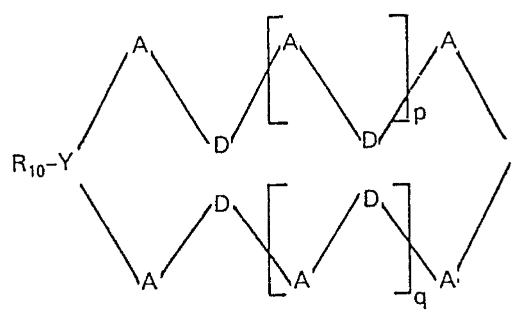

IIa

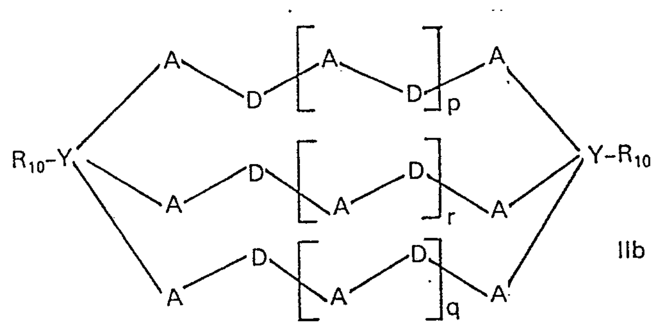

IIb

dans lesquelles:

- Y représente N ou P
- A représente un groupement alkylène ayant de 1 à 3 atomes de carbone
- D représente O, S ou N-$R_{11}$ où $R_{11}$ représente un radical alkyle ayant de 1 à 6 atomes de carbone
- $R_{10}$ représente un radical alkyle ayant de 1 à 6 atomes de carbone, p, q et r identiques ou différents sont des nombres entiers compris entre 1 et 5.

Selon un quatrième mode de mise en œuvre particulier de l'invention, on utilise un mélange d'au moins deux des composés complexant définis ci-dessus.

Les dichlorobenzènes mis en œuvre dans le procédé selon l'invention sont le dichloro-1,2 benzène, le dichloro-1,3 benzène et le dichloro-1,4 benzène.

Les trichlorobenzènes concernés sont le trichloro-1,2,3 benzène, le trichloro-1,2,4 benzène, et le trichloro-1,3,5 benzène.

Les tétrachlorobenzènes sont le tétrachloro-1,2,4,5 benzène, le tétrachloro-1,2,3,5 benzène et le tétrachloro-1,2,3,4 benzène.

Bien que cela ne soit pas complètement démontré, il apparaît que l'ivention repose sur le fait que composé complexant forme avec l'amidure alcalin et/ou l'alcoolate alcalin un complexe qui permet l'arrachement d'un hydrogène sur un polychlorobenzène lequel donne un carbanion. Ce carbanion du fait de la complexation du cation alcalin est soluble dans le milieu ce qui lui permet d'arracher alors un chlore d'une autre molécule de polychlorobenzène, la réaction se reproduisant selon le même schéma avec les autres espèces présentes dans le milieu réactionnel.

C'est ainsi, par exemple, que lorsque l'on met en œuvre un mélange pentachlorobenzène-trichloro-1,2,4 benzène, l'anion amidure ou alcoolate arrache un hydrogène au trichlorobenzène pour donner les trois carbanions suivants:

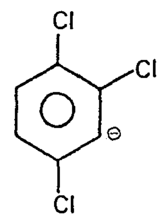

CI

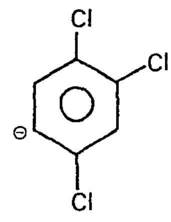

CII

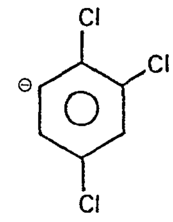

CIII

en proportions variables suivant l'acidité de l'hydrogène arraché. C'est le carbanion CI qui sera ici majoritaire, mais ce sera le carbanion CIII qui sera le plus réactif dans l'étape suivante d'arrachement d'un atome de chlore du pentachlorobenzène.

L'action du carbanion CIII sur le pentachlorobenzène donne principalement le carbanion:

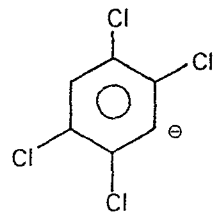

CIV

résultant de l'arrachement du chlore 3 du pentachlorobenzène (d'autres carbanions se forment par arrachement d'un atome de chlore sur les autres sites, mais en proportions plus faibles) et le tétrachloro-1,2,3,5:

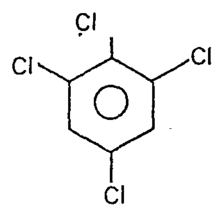

Ainsi, à partir d'un trichlorobenzène et d'un pentachlorobenzène, on a obtenu deux tétrachlorobenzènes, selon le schéma réactionnel suivant:

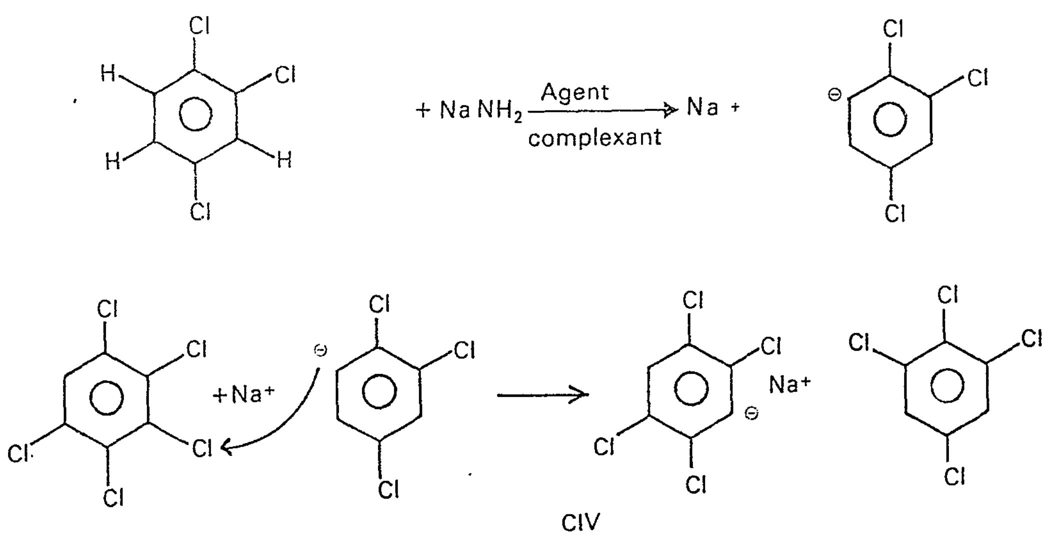


CIV

c'est en ce sens que l'on parle de réarrangement.

D'autre part, le trichloro-1,2,4 benzène peut se transformer en trichloro-1,3,5 benzène. Lorsque l'on mélange, dans les conditions de l'invention, un tétrachlorobenzène (comme par exemple le tétrachloro-1,2,4,5 benzène) avec le trichloro-1,2,4 benzène, on observe une disparition de ce dernier au profit du trichloro-1,3,5 benzène. Bien que cela ne soit pas clairement démontré, il semblerait qu'une molécule de tétrachlorobenzène perde un proton pour donner un carbanion, lequel va arracher un chlore à une autre molécule de tétrachlorobenzène conduisant ainsi à une molécule de pentachlorobenzène et de trichloro-1,2,4 benzène.

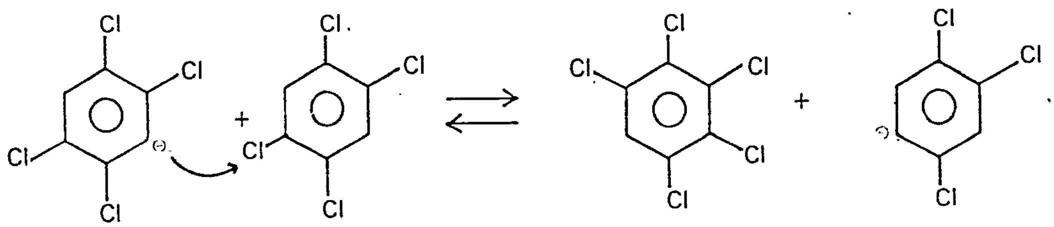

Comme cela a été dit précédemment, cette réaction est reversible. Mais d'autres réactions sont également possibles dans ces conditions opératoires et on observe tout d'abourd l'apparition de tétrachloro-1,2,3,5 benzène puis de trichloro-1,3,5 benzène. La présence en grande quantité de trichloro-1,2,4 benzène favorise la recombinaison de celui-ci avec le pentachlorobenzène, diminuant ainsi sa concentration. Le bilan de la réaction se traduit alors par une isomérisation du trichloro-1,2,4 benzène en trichloro-1,3,5 benzène en présence de tétrachlorobenzène.

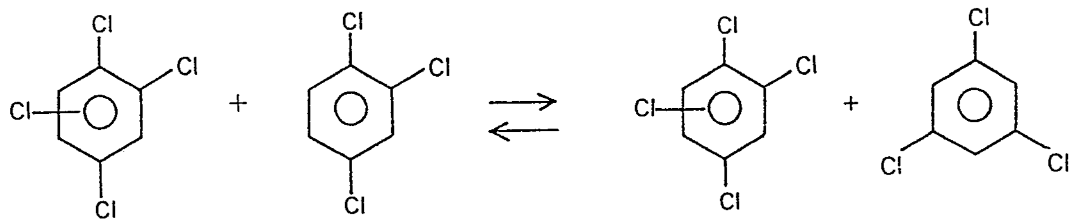

De la même façon lorsque l'on [illegible] en œuvre [illegible] comme produit de départ, le tétrachloro-1,2,3,4 benzène, on observe une transformation quasi quantitative (de l'ordre de 90%) en isomères-1,2,3,5 et 1,2,4,5 et en trichloro-1,2,4 et 1,3,5 benzène et en pentachlorobenzène.

Selon un mode de réalisation préférentiel de l'invention, on utilise un agent séquestrant de formule (I) dans laquelle $R_1$, $R_2$, $R_3$ et $R_4$ représentent un atome d'hydrogène ou un radical méthyle, $R_5$ et n ayant la signification précédente.

Parmi ces derniers, on préfère encore plus particulièrement mettre en œuvre les agents séquestrants pour lesquels n est supérieur ou égal à 0 et inférieur ou égal à 6 et pour lesquels $R_5$ représente un radical alkyle ayant de 1 à 4 atomes de carbone.

On peut citer:
- la tris(oxa-3 butyl)amine de formule:
$N(CH_2\text{-}CH_2\text{-}O\text{-}CH_3)_3$
- La tris(dioxa-3,6 heptyl)amine de formule:
$N(CH_2\text{-}CH_2\text{-}O\text{-}CH_2\text{-}CH_2\text{-}O\text{-}CH_3)_3$
- la tris(trioxa-3,6,9 décyl)amine de formule:
$N(CH_2\text{-}CH_2\text{-}O\text{-}CH_2CH_2\text{-}O\text{-}CH_2\text{-}CH_2\text{-}O\text{-}CH_3)_3$
- la tris(dioxa-3,6 octyl)amine de formule:
$N(CH_2\text{-}CH_2\text{-}O\text{-}CH_2\text{-}CH_2\text{-}O\text{-}C_2H_5)_3$
- la tris(trioxa-3,6,9 undécyl)amine de formule:
$N(CH_2\text{-}CH_2\text{-}O\text{-}CH_2CH_2\text{-}O\text{-}CH_2\text{-}CH_2\text{-}O\text{-}C_2H_5)_3$
- la tris(dioxa-3,6 nonyl)amine de formule:
$N(CH_2\text{-}CH_2\text{-}O\text{-}CH_2\text{-}CH_2\text{-}O\text{-}C_3H_7)_3$
- la tris(trioxa-3,6,9 dodécyl)amine de formule:
$N(CH_2\text{-}CH_2\text{-}O\text{-}CH_2\text{-}CH_2\text{-}O\text{-}CH_2\text{-}CH_2\text{-}O\text{-}C_3H_7)_3$
- la tris(di[illegible])amine de fo[illegible] [illegible]oxa-3,6 décyl[illegible] formule:
$N(CH_2\text{-}CH_2\text{-}O\text{-}CH_2\text{-}CH_2\text{-}O\text{-}C_4H_9)_3$
- la tris(trioxa-3,6,9 tridécyl)amine de formule:
$N(CH_2\text{-}CH_2\text{-}O\text{-}CH_2\text{-}CH_2\text{-}O\text{-}CH_2\text{-}CH_2\text{-}O\text{-}C_4H_9)_3$
- la tris (tetra-oxa-3,6,9,12 tridecyl) amine de formule:
$N\text{-}[CH_2\text{-}CH_2\text{-}O(CH_2\text{-}CH_2\text{-}O)_3\text{-}CH_3]_3$
- La tris(hexa-oxa-3,6,9,12,15,18 nonadecyl)amine de formule:
$N\text{-}[CH_2\text{-}CH_2\text{-}O\text{-}(CH_2\text{-}CH_2\text{-})_5CH_3]_3$
- la tris(dioxa-3,6 méthyl-4 heptyl) amine de formule:
$N\text{-}[CH_2\text{-}CH_2\text{-}OCH\text{-}(CH_3)\text{-}CH_2\text{-}O\text{-}CH_3]_3$
- La tris(dioxa-3,6 diméthyl-2,4 heptyl) amine de formule:
$N\text{-}[CH_2CH\text{-}(CH_3)\text{-}OCH(CH_3)\text{-}CH_2\text{-}O\text{-}CH_3]_3$.

Les amines utilisées dans le procédé selon l'invention sont connues en tant que telles dans l'art antérieur. C'est ainsi que le brevet français 1.302.365 cite l'obtention des amines tertiaires $N(CH_2\text{-}CH_2\text{-}O\text{-}CH_3)_3$ et $N(CH_2\text{-}CH_2\text{-}O\text{-}CH_2\text{-}CH_2\text{-}O\text{-}CH_3)_3$ comme sous produits de la synthèse des amines primaires et secondaires correspondantes, ces amines primaires et secondaires étant des produits intéressants comme intermédiaires en vue de la synthèse de substances pharmaceutiques, comme inhibiteurs de corrosion, comme intermédiaires en vue de la synthèse de produits chimiques intéressants en agriculture et comme émulsifiants. Il n'est pas inutile de souligner que le domaine d'application des composés obtenus dans le brevet 1.302.365 précité simulta-

ne-1,2,4,5 (0,05 mole) et 9 grammes de trichloro-1,2,4 benzène (0,05 mole), 30 grammes de toluène, 0,8 gramme de tris(dioxa-3,6 heptyl)amine et 2 grammes d'amidure de sodium (0,05 mole). Après une heure sous agitation à 30°C, le mélange de polychlorobenzènes se compose de:

| Polychlorobenzènes | Pourcentage |
|---|---|
| trichloro-1,3,5 benzène | 12,3 |
| trichloro-1,2,4 benzène | 43 |
| tétrachloro-1,2,4,5 benzène | 16 |
| tétrachloro-1,2,3,5 benzène | 23 |
| tétrachloro-1,2,3,4 benzène | 3,8 |
| pentachlorobenzène | 1,8 |

Exemple no 9:
Dans un réacteur de 150 ml muni d'un réfrigérant et d'une cuve de refroidissement, on introduit sous azote (anhydre) 43,6 g de tétrachloro-1,2,4,5 benzène (0,2 mole), 9 grammes de trichloro-1,2,4 benzène (0,05 mole), 150 g de chlorobenzène, 1,6 g de tris(dioxa-3,6 heptyl)amine (0,005 mole), 2 g d'amidure de sodium (0,05 mole).

Après 30 minutes sous agitation à 10°C, la composition du mélange de polychlorobenzènes est:

| Polychlorobenzènes | Pourcentage |
|---|---|
| trichloro-1,3,5 benzène | 10,6 |
| trichloro-1,2,4 benzène | 32 |
| tétrachloro-1,2,3,5 benzène | 19 |
| tétrachloro-1,2,4,5 benzène | 28 |
| tétrachloro-1,2,3,4 benzène | 4,4 |
| pentachlorobenzène | 6 |

Exemple no 10:
Dans un réacteur de 50 ml équipé d'un réfrigérant et d'une agitation magnétique, on introduit sous azote (anhydre):

- 0,05 mole de trichloro-1,2,4 benzène soit 9,07 g
- 0,02 mole de tétrachloro-1,2,4,5 benzène soit 4,3 g
- 0,02 mole de tétrachloro-1,2,3,5 benzène soit 4,3 g

puis on rajoute 10 grammes de chlorobenzène anhydre.

Le mélange est agité à température ambiante de 18°C.

On ajoute 0,0025 mole de l'éther couronne «Dicyclohexyl 18 C-6» de formule:

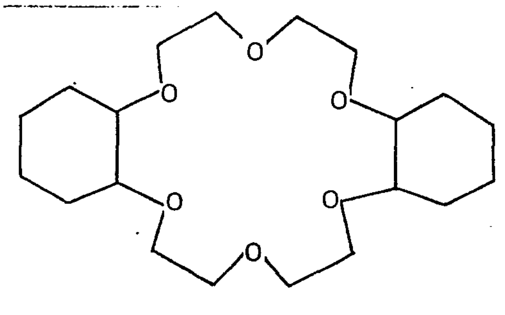

soit 0,93 g et un mélange d'amidure de sodium dans le toluène (50% - 50%) soit environ 0,02 mole d'amidure de sodium.

On observe une élévation de température de 18°C à 24°C.

Après 4 heures de réaction sous agitation le mélange polychlorobenzène obtenu se compose de:

| Polychlorobenzène | Pourcentage |
|---|---|
| trichloro-1,3,5 benzène | 14 |
| trichloro-1,2,4 benzène | 53 |
| tétrachloro-1,2,3,5 benzène | |
| tétrachloro-1,2,4,5 benzène | 27,8 |
| tétrachloro-1,2,3,4 benzène | 3,8 |
| pentachlorobenzène | 1,1 |

Exemple no 11:
Dans un réacteur de 50 ml équipé d'un réfrigérant et d'une agitation magnétique, on introduit sous azote (anhydre):

- 0,05 mole de trichloro-1,2,4 benzène soit 9,07 g
- 0,04 mole de tétrachloro-1,2,4,5 benzène soit 8,6 g

puis on rajoute 10 g de chlorobenzène anhydre.

Le mélange est agité à température ambiante de 19°C.

On ajoute 0,005 mole d'un composé bicyclique de formule:

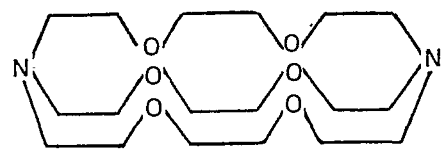

et un mélange d'amidure de sodium dans le toluène (50% - 50%) soit environ 0,02 mole d'amidure de sodium.

Après 1 heure de réaction sous agitation le mélange polychlorobenzène obtenu se compose de:

| Polychlorobenzène | Pourcentage |
|---|---|
| trichloro-1,3,5 benzène | 27,6 |
| trichloro-1,2,4 benzène | 36,8 |
| tétrachloro-1,2,3,5 benzène | |
| tétrachloro-1,2,4,5 benzène | 31,3 |
| tétrachloro-1,2,3,4 benzène | 2,7 |
| pentachlorobenzène | 1,6 |

Exemple no 12:
Dans un réacteur de 250 ml double enveloppe, agité par barreau magnétique, on introduit sous azote (anhydre)

- 36,3 g de trichloro-1,2,4 benzène
- 6,4 g de tris(dioxa-3,6 heptyl)amine
- 6 g de Na $NH_2$
- 16,2 g de tétrachloro-1,2,4,5 benzène

On maintient une température de 17°C pendant 2 heures.

Le mélange réactionnel est ensuite repris par 200 ml d'eau et 50 ml de dichlorométhane. La couche organique est ensuite analysée:

nément aux amines utilisées dans le procédé objet de la présente demande est totalement étranger au domaine de l'invention.

Les polyéthers macrocycliques qui peuvent être mis en œuvre dans le procédé selon l'invention sont connus sous l'appellation générale d'«éthers couronnes» et sont décrits dans le brevet fançais 69.43879 publié sous le numéro 2.026.481.

On peut citer comme exemples d'éthers couronnes pouvant être utilisés selon l'invention:

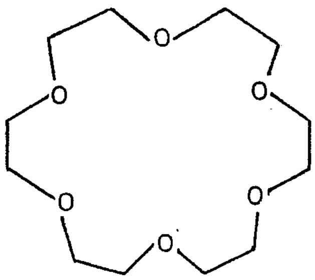

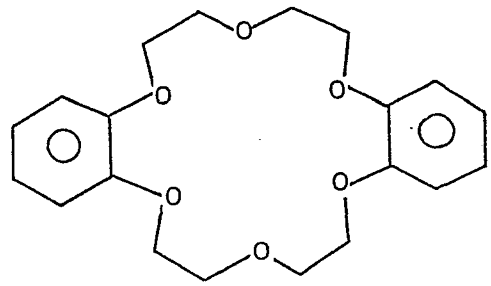

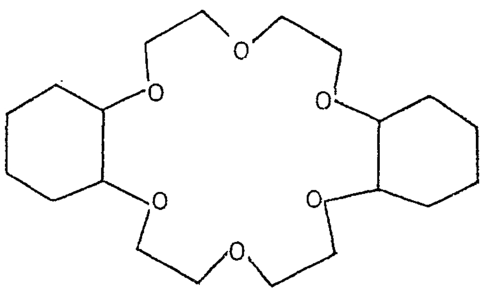

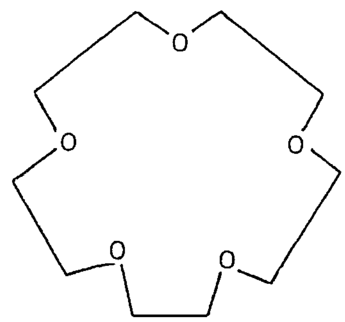

Les composés macroxycliques et bicycliques sont décrits dans le brevet français 70.21079 publié sous le numéro 2.052.947. On peut citer comme exemples de tels composés concernant la mise en œuvre du procédé selon l'invention:

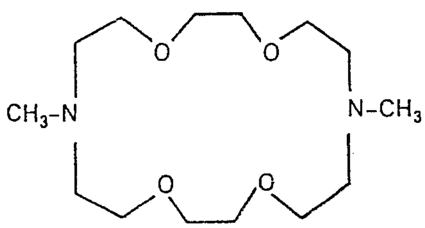

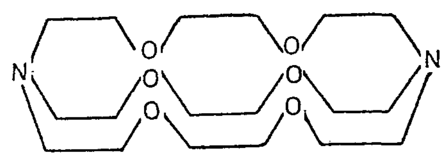

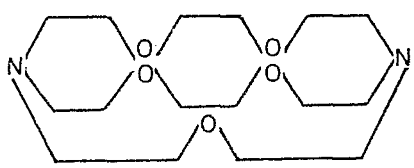

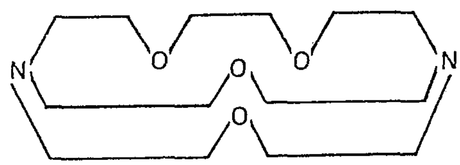

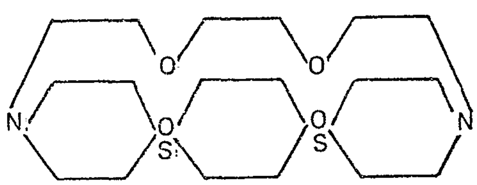

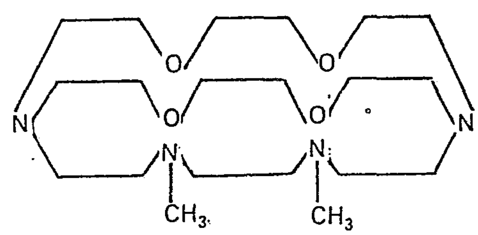

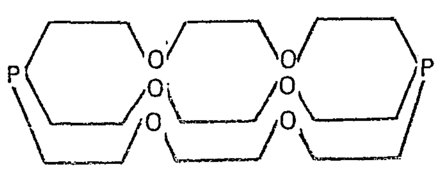

ne-1,2,4,5 (0,05 mole) et 9 grammes de trichloro-1,2,4 benzène (0,05 mole), 30 grammes de toluène, 0,8 gramme de tris(dioxa-3,6 heptyl)amine et 2 grammes d'amidure de sodium (0,05 mole). Après une heure sous agitation à 30°C, le mélange de polychlorobenzènes se compose de:

| Polychlorobenzènes | Pourcentage |
|---|---|
| trichloro-1,3,5 benzène | 12,3 |
| trichloro-1,2,4 benzène | 43 |
| tétrachloro-1,2,4,5 benzène | 16 |
| tétrachloro-1,2,3,5 benzène | 23 |
| tétrachloro-1,2,3,4 benzène | 3,8 |
| pentachlorobenzène | 1,8 |

Exemple no 9:
Dans un réacteur de 150 ml muni d'un réfrigérant et d'une cuve de refroidissement, on introduit sous azote (anhydre) 43,6 g de tétrachloro-1,2,4,5 benzène (0,2 mole), 9 grammes de trichloro-1,2,4 benzène (0,05 mole), 150 g de chlorobenzène, 1,6 g de tris(dioxa-3,6 heptyl)amine (0,005 mole), 2 g d'amidure de sodium (0,05 mole).

Après 30 minutes sous agitation à 10°C, la composition du mélange de polychlorobenzènes est:

| Polychlorobenzènes | Pourcentage |
|---|---|
| trichloro-1,3,5 benzène | 10,6 |
| trichloro-1,2,4 benzène | 32 |
| tétrachloro-1,2,3,5 benzène | 19 |
| tétrachloro-1,2,4,5 benzène | 28 |
| tétrachloro-1,2,3,4 benzène | 4,4 |
| pentachlorobenzène | 6 |

Exemple no 10:
Dans un réacteur de 50 ml équipé d'un réfrigérant et d'une agitation magnétique, on introduit sous azote (anhydre):

- 0,05 mole de trichloro-1,2,4 benzène soit 9,07 g
- 0,02 mole de tétrachloro-1,2,4,5 benzène soit 4,3 g
- 0,02 mole de tétrachloro-1,2,3,5 benzène soit 4,3 g

puis on rajoute 10 grammes de chlorobenzène anhydre.

Le mélange est agité à température ambiante de 18°C.

On ajoute 0,0025 mole de l'éther couronne «Dicyclohexyl 18 C-6» de formule:

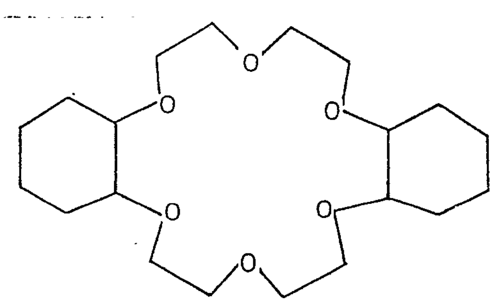

soit 0,93 g et un mélange d'amidure de sodium dans le toluène (50% - 50%) soit environ 0,02 mole d'amidure de sodium.

On observe une élévation de température de 18°C à 24°C.

Après 4 heures de réaction sous agitation le mélange polychlorobenzène obtenu se compose de:

| Polychlorobenzène | Pourcentage |
|---|---|
| trichloro-1,3,5 benzène | 14 |
| trichloro-1,2,4 benzène | 53 |
| tétrachloro-1,2,3,5 benzène | |
| tétrachloro-1,2,4,5 benzène | 27,8 |
| tétrachloro-1,2,3,4 benzène | 3,8 |
| pentachlorobenzène | 1,1 |

Exemple no 11:
Dans un réacteur de 50 ml équipé d'un réfrigérant et d'une agitation magnétique, on introduit sous azote (anhydre):

- 0,05 mole de trichloro-1,2,4 benzène soit 9,07 g
- 0,04 mole de tétrachloro-1,2,4,5 benzène soit 8,6 g

puis on rajoute 10 g de chlorobenzène anhydre.

Le mélange est agité à température ambiante de 19°C.

On ajoute 0,005 mole d'un composé bicyclique de formule:

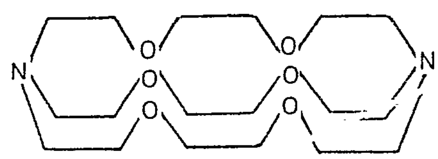

et un mélange d'amidure de sodium dans le toluène (50% - 50%) soit environ 0,02 mole d'amidure de sodium.

Après 1 heure de réaction sous agitation le mélange polychlorobenzène obtenu se compose de:

| Polychlorobenzène | Pourcentage |
|---|---|
| trichloro-1,3,5 benzène | 27,6 |
| trichloro-1,2,4 benzène | 36,8 |
| tétrachloro-1,2,3,5 benzène | |
| tétrachloro-1,2,4,5 benzène | 31,3 |
| tétrachloro-1,2,3,4 benzène | 2,7 |
| pentachlorobenzène | 1,6 |

Exemple no 12:
Dans un réacteur de 250 ml double enveloppe, agité par barreau magnétique, on introduit sous azote (anhydre)

- 36,3 g de trichloro-1,2,4 benzène
- 6,4 g de tris(dioxa-3,6 heptyl)amine
- 6 g de Na $NH_2$
- 16,2 g de tétrachloro-1,2,4,5 benzène

On maintient une température de 17°C pendant 2 heures.

Le mélange réactionnel est ensuite repris par 200 ml d'eau et 50 ml de dichlorométhane. La couche organique est ensuite analysée:

| Polychlorobenzènes | Pourcentage |
|---|---|
| trichloro-1,3,5 benzène | 32,6 |
| trichloro-1,2,4 benzène | 42,8 |
| tétrachloro-1,2,4,5 benzène | 17 |
| tétrachloro-1,2,3,5 benzène | 12,5 |
| tétrachloro-1,2,3,4 benzène | 3 |
| pentachlorobenzène | 2,1 |

**Revendications**

1. Procédé d'isomérisation et/ou de réarrangement de polychlorobenzène caractérisé en ce que l'on met en réaction, d'une part:
- au moins un tétrachlorobenzène éventuellement en mélange avec au moins un trichlorobenzène et/ou le pentachlorobenzène et/ou l'hexachlorobenzène
- ou le pentachlorobenzène en mélange avec au moins un trichlorobenzène et/ou l'hexachlorobenzène
- ou l'hexachlorobenzène en mélange avec au moins un trichlorobenzène et/ou un dichlorobenzène

et, d'autre part, au moins un amidure alcalin et/ou un alcoolate alcalin en présence d'au moins un composé complexant le cation de l'amidure et/ou l'alcoolate.

2. Procédé selon la revendication 1, caractérisé en ce que le composé complexant est un agent séquestrant de formule:

$$N-[CHR_1-CHR_2-O-(CHR_3-CHR_4-O)_n-R_5]_3 \quad (I)$$

dans laquelle n est un nombre entier supérieur ou égal à 0 et inférieur ou égal à 10 ($0 \leqq n \leqq 10$), $R_1$, $R_2$, $R_3$ et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $-C_mH_{2m}-\emptyset$ ou $C_mH_{2m+1}-\emptyset-$, où m est compris entre 1 et 12 ($1 \leqq m \leqq 12$).

3. Procédé selon la revendication 2 caractérisé en ce que dans la formule (I) $R_1$, $R_2$, $R_3$ et $R_4$ représentent un atome d'hydrogène ou un radical méthyle.

4. Procédé selon l'une quelconque des revendications 2 et 3, caractérisé en ce que dans la formule (I) n est un nombre entier supérieur ou égal à 0 et inférieur ou égal à 6.

5. Procédé selon l'une quelconque des revendications 2 à 4, caractérisé en ce que dans la formule (I) $R_5$ représente un radical alkyle ayant de 1 à 4 atomes de carbone.

6. Procédé selon la revendication 2, caractérisé en ce que l'agent séquestrant de formule (I) est la tris(dioxa-3,6 heptyl) amine de formule:

$$N-(CH_2-CH_2-O-CH_2-CH_2-O-CH_3)_3$$

7. Procédé selon la revendication 2 caractérisé en ce que l'agent séquestrant de formule (I) est la tris(dioxa-3,6 octyl) amine de formule:

$$N-(CH_2-CH_2-O-CH_2-CH_2-O-C_2H_5)_3$$

8. Procédé selon la revendication 1, caractérisé en ce que le composé complexant est un polyéther macrocyclique ayant de 15 à 30 atomes dans le cycle et constitué de 5 à 10 unités -O-X dans lesquelles X est soit $-CHR_6-CHR_7-$ soit $-CHR_6-CHR_8-CR_9R_7-$, $R_6$, $R_7$, $R_8$ et $R_9$ identiques ou différents étant un atome d'hydrogène ou un radical alkyle ayant de 1 à 4 atomes de carbone, un des X pouvant être $-CHR_6-CHR_8-CR_9R_7-$ quand les unités -O-X comprennent le groupement $-O-CHR_6-CHR_7-$

9. Procédé selon la revendication 8, caractérisé en ce que le polyéther macrocyclique est choisi parmi le groupe comprenant:

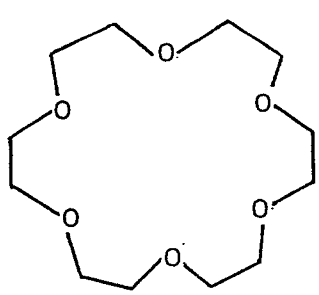

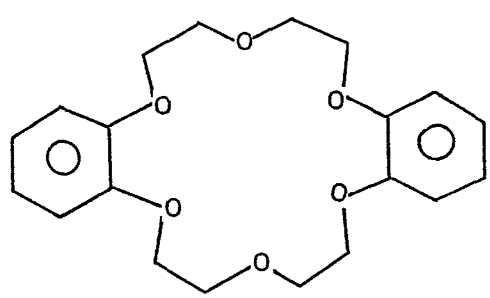

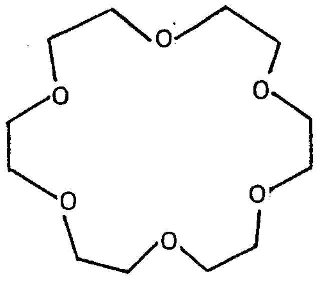

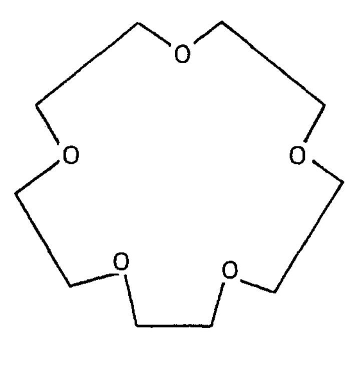

12. Procédé selon l'une quelconque des revendications précédentes caractérisé en ce qu'on opère en présence d'un solvant aromatique.

13. Procédé selon la revendication 12 caractérisé en ce que le solvant est choisi parmi le groupe comprenant le chlorobenzène, le benzène et le toluène.

14. Procédé selon l'une quelconque des revendications précédentes, caractérisé en ce que l'amidure alcalin est l'amidure de lithium, de potassium ou de sodium.

15. Procédé selon l'une quelconque des revendications précédentes, caractérisé en ce que l'alcoolate alcalin est choisi parmi le groupe comprenant les composés $RO\text{-}M^+$ ou $M^-$ représente le sodium ou le potassium et où R représente un radical alkyle contenant de 1 à 6 atomes de carbone.

16. Procédé selon l'une quelconque des revendications précédentes caractérisé en ce qu'on opère à une température comprise entre −20°C et 60°C.

17. Procédé selon l'une quelconque des revendications précédentes, caractérisé en ce que le rapport molaire de l'amidure alcalin à l'agent séquestrant de formule (I) est compris entre 0,005 et 0,5.

18. Procédé selon l'une quelconque des revendications précédentes, caractérisé en ce que le rapport de l'amidure alcalin au(x) polychlorobenzène(s) de départ est compris entre 1 et 0,05.

19. Procédé selon l'une quelconque des revendications précédentes pour la préparation du trichloro-1,2,5 benzène caractérisé en ce que l'on met en réaction au moins un tétrachlorobenzène éventuellement en mélange avec le trichloro-1,2,3 benzène et/ou le trichloro-1,2,4 benzène et/ou le pentachlorobenzène et/ou l'hexachlorobenzène.

20. Procédé selon la revendication 19, caractérisé en ce que l'on met en réaction au moins un tétrachlorobenzène en mélange avec le trichloro-1,2,4 benzène.

21. Procédé selon la revendication 19, caractérisé en ce que l'on met en réaction le mélange tétrachloro-1,2,4,5 benzène et trichloro-1,2,4 benzène.

22. Procédé selon l'une quelconque des revendications 19 et 20, caractérisé en ce que le rapport molaire tétrachloro au trichloro est compris entre 0,1 et 10.

## Patentansprüche

1. Verfahren zur Isomerisierung und/oder Umlagerung von Polychlorbenzolen, dadurch gekennzeichnet, dass man einerseits

- wenigstens ein Tetrachlorbenzol, gegebenenfalls im Gemisch mit wenigstens einem Trichlorbenzol und/oder dem Pentachlorbenzol und/oder dem Hexachlorbenzol
- oder das Pentachlorbenzol im Gemisch mit wenigstens einem Trichlorbenzol und/oder dem Hexachlorbenzol
- oder Hexachlorbenzol im Gemisch mit wenigstens einem Trichlorbenzol und/oder Dichlorbenzol

und andererseits wenigstens ein Alkaliamid und/oder Alkalialkoholat in Gegenwart wenigstens eines Komplexbildners für das Kation des Amids und/oder Alkoholats umsetzt.

2. Verfahren nach Anspruch 1, dadurch gekennzeichnet, dass der Komplexbildner ein Sequestrierungsmittel der Formel

$$N\text{-}[CHR_1\text{-}CHR_2\text{-}O\text{-}(CHR_3\text{-}CHR_4\text{-}O)_n\text{-}R_5]_3 \quad (I)$$

ist, in der n eine ganze Zahl grösser oder gleich 0 und kleiner oder gleich 10 ($0 \leq n \leq 10$) ist, $R_1$, $R_2$, $R_3$, $R_4$ gleich oder verschieden ein Wasserstoffatom oder einen Alkylrest mit 1 bis 4 Kohlenstoffatomen bedeuten und $R_5$ einen Alkyl- oder Cycloalkylrest mit 1 bis 12 Kohlenstoffatomen, einen Phenylrest oder einen Rest $-C_mH_{2m}-\emptyset$ oder $C_mH_{2m+1}-\emptyset-$ bedeutet, wobei m zwischen 1 und 12 liegt ($1 \leq m \leq 12$).

3. Verfahren nach Anspruch 2, dadurch gekennzeichnet, dass in der Formel (I) $R_1$, $R_2$, $R_3$ und $R_4$ ein Wasserstoffatom oder einen Methylrest bedeuten.

4. Verfahren nach irgendeinem der Ansprüche 2 und 3, dadurch gekennzeichnet, dass in Formel (I) n eine ganze Zahl grösser oder gleich Null und kleiner oder gleich 6 ist.

5. Verfahren nach irgendeinem der Ansprüche 2 bis 4, dadurch gekennzeichnet, dass in der Formel I $R_5$ ein Alkylrest mit 1 bis 4 Kohlenstoffatomen ist.

6. Verfahren nach Anspruch 2, dadurch gekennzeichnet, dass das Sequestrierungsmittel der Formel (I) das Tris(dioxa-3,6-heptyl)amin der Formel

$$N\text{-}(CH_2\text{-}CH_2\text{-}O\text{-}CH_2\text{-}CH_2\text{-}O\text{-}CH_3)_3$$

ist.

7. Verfahren nach Anspruch 2, dadurch gekennzeichnet, dass das Sequestrierungsmittel der Formel (I) das Tris(dioxa-3,6-octyl)amin der Formel

$$N\text{-}(CH_2\text{-}CH_2\text{-}O\text{-}CH_2\text{-}CH_2\text{-}O\text{-}C_2H_5)_3$$

ist.

8. Verfahren nach Anspruch 1, dadurch gekennzeichnet, dass der Komplexbildner ein macrocyclischer Polyäther mit 15 bis 30 Atomen im Ring ist und aus 5 bis 10 -O-X-Einheiten besteht, in denen X entweder $-CHR_6-CHR_7$ oder $-CHR_6-CHR_8-CR_9R_7-$ ist, wobei $R_6$, $R_7$, $R_8$ und $R_9$ gleich oder verschieden ein Wasserstoffatom oder einen Alkylrest mit 1 bis 4 Kohlenstoffatomen bedeuten und einer der Reste X auch $-CHR_6-CHR_8-CR_9R_7-$ sein kann, wenn die -O-X-Einheiten die $-O-CHR_6-CHR_7-$ Gruppe enthalten.

9. Verfahren nach Anspruch 8, dadurch gekennzeichnet, dass der macrocyclische Polyäther aus der folgenden Gruppe ausgewählt ist:

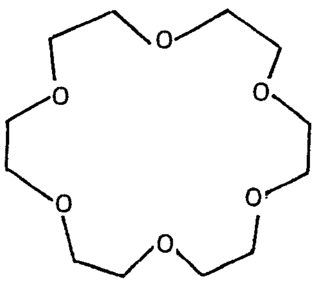

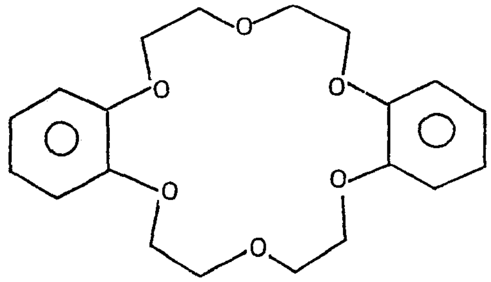

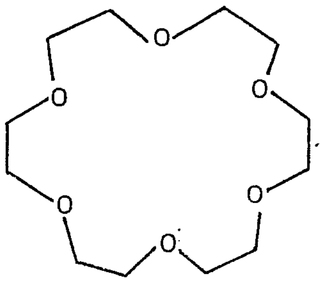

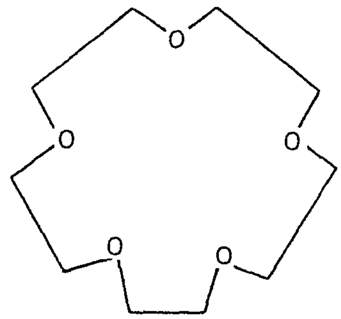

10. Verfahren nach Anspruch 1, dadurch gekennzeichnet, dass der Komplexbildner eine macrocyclische oder bicyclische Verbindung der allgemeinen Formeln IIa oder IIb ist,

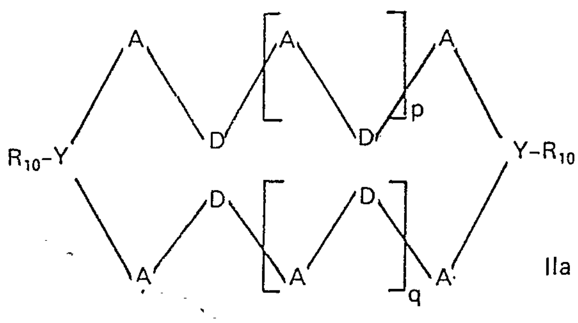

IIa

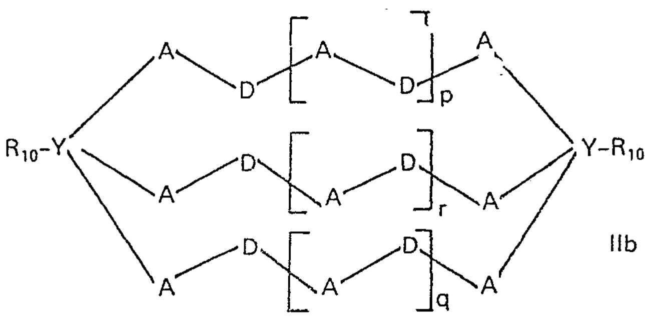

IIb

in denen

-Y die Bedeutung von N oder P hat

- A eine Alkylengruppe mit 1 bis 3 Kohlenstoffatomen bedeutet

- D gleich 0, S oder N-$R_{11}$ ist, wobei $R_{11}$ ein Alkylrest mit 1 bis 6 Kohlenstoffatomen ist

- $R_{10}$ einen Alkylrest mit 1 bis 6 Kohlenstoffatomen bedeutet; und p, q, r identisch oder verschieden ganze Zahlen zwischen 1 und 5 sind.

11. Verfahren nach Anspruch 10, dadurch gekennzeichnet, dass die macrocyclische oder bicyclische Verbindung aus der folgenden Gruppe ausgewählt ist:

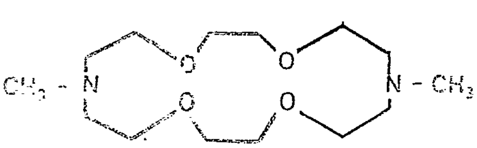

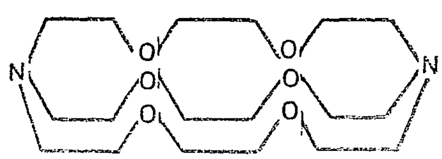

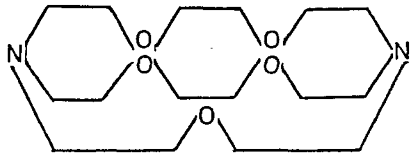

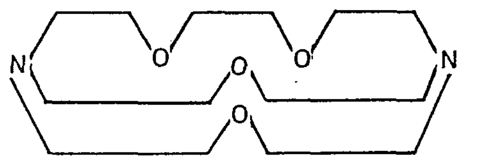

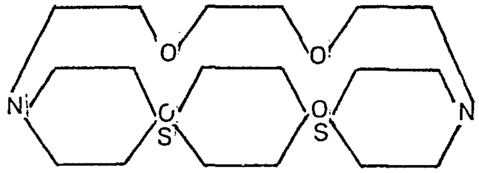

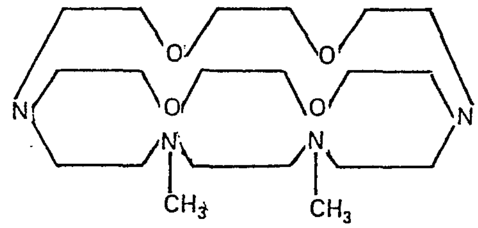

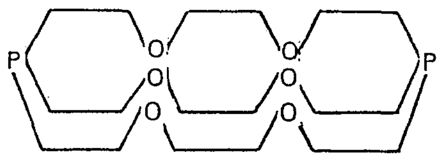

12. Verfahren nach irgendeinem der vorangehenden Ansprüche, dadurch gekennzeichnet, dass man in Gegenwart eines aromatischen Lösungsmittels arbeitet.

13. Verfahren nach Anspruch 12, dadurch gekennzeichnet, dass das Lösungsmittel aus der Gruppe von Chlorbenzol, Benzol und Toluol ausgewählt ist.

14. Verfahren nach irgendeinem der vorangehenden Ansprüche, dadurch gekennzeichnet, dass das Alkaliamid das Amid von Lithium, Kalium oder Natrium ist.

15. Verfahren nach irgendeinem der vorangehenden Ansprüche, dadurch gekennzeichnet, dass das Alkalialkoholat aus der Gruppe der Verbindungen $RO^-M^+$ ausgewählt ist, in der $M^+$ Natrium oder Kalium bedeutet, und wo R einen Alkylrest mit 1 bis 6 Kohlenstoffatomen bedeutet.

16. Verfahren nach irgendeinem der vorangehenden Ansprüche, dadurch gekennzeichnet, dass man bei einer Temperatur zwischen −20 und +60°C arbeitet.

17. Verfahren nach irgendeinem der vorangehenden Ansprüche, dadurch gekennzeichnet, dass das Molverhältnis des Alkaliamids zum Sequestrierungsmittel der Formel I 0,005 bis 0,5 beträgt.

18. Verfahren nach irgendeinem der vorangehenden Ansprüche, dadurch gekennzeichnet, dass das Verhältnis des Alkaliamids zu dem oder den eingesetzten Polychlorbenzol(en) 1 bis 0,05 beträgt.

19. Verfahren nach irgendeinem der vorangehenden Ansprüche für die Herstellung von 1,2,5-Trichlorbenzol, dadurch gekennzeichnet, dass man wenigstens ein Tetrachlorbenzol, gegebenenfalls im Gemisch mit 1,2,3-Trichlorbenzol und/oder dem 1,2,4-Trichlorbenzol und/oder dem Pentachlorbenzol und/oder Hexachlorbenzol umsetzt.

20. Verfahren nach Anspruch 19, dadurch gekennzeichnet, dass man wenigstens ein Tetrachlorbenzol im Gemisch mit dem 1,2,4-Trichlorbenzol umsetzt.

21. Verfahren nach Anspruch 19, dadurch gekennzeichnet, dass man das Gemisch von 1,2,4,5-Tetrachlorbenzol und 1,2,4-Trichlorbenzol umsetzt.

22. Verfahren nach einem der Ansprüche 19 und 20, dadurch gekennzeichnet, dass das Molverhältnis von Tretrachlorbenzol zum Trichlorbenzol 0,1 bis 10 beträgt.

## Claims

1. Process for the isomerisation and/or rearrangement of polychlorobenzenes, characterised in that, on the one hand:

- at least one tetrachlorobenzene, if appropriate mixed with at least one trichlorobenzene and/or pentachlorobenzene and/or hexachlorobenzene,
- or pentachlorobenzene mixed with at least one trichlorobenzene and/or hexachlorobenzene,
- or hexachlorobenzene mixed with at least one trichlorobenzene and/or one dichlorobenzene,

and, on the other hand, at least one alkali metal amide and/or one alkali metal alcoholate, are reacted in the presence of at least one compound forming a complex with the cation of the amide and/or the alcoholate.

2. Process according to Claim 1, characterised in that the complexing compound is a sequestering agent of the formula:

$$N\text{-}[CHR_1\text{-}CHR_2\text{-}O\text{-}(CHR_3\text{-}CHR_4\text{-}O)_n\text{-}R_5]_3 \quad (I),$$

in which n is an integer greater than or equal to O and less than or equal to 10 ($0 \leq n \leq 10$), $R_1$, $R_2$, $R_3$ and $R_4$, which are identical or different, represent a hydrogen atom or an alkyl radical having from 1 to 4 carbon atoms and $R_5$ represents an alkyl or cycloalkyl radical having from 1 to 12 carbon atoms, a phenyl radical or a radical $-C_mH_{2m}-\varnothing$ or

$C_mH_{2m+1}$-Ø-, in which m is between 1 and 12 ($1 \leq m \leq 12$).

3. Process according to Claim 2, characterised in that, in the formula (I), $R_1$, $R_2$, $R_3$ and $R_4$ represent a hydrogen atom or a methyl radical.

4. Process according to either one of Claims 2 and 3, characterised in that, in the formula (I), n is an integer greater than or equal to 0 and less than or equal to 6.

5. Process according to any one of Claims 2 to 4, characterised in that, in the formula (I), $R_5$ represents an alkyl radical having from 1 to 4 carbon atoms.

6. Process according to Claim 2, characterised in that the sequestering agent of the formula (I) is tris(3,6-dioxaheptyl)-amine of the formula:

$N-(CH_2-CH_2-O-CH_2-CH_2-O-CH_3)_3$

7. Process according to Claim 2, characterised in that the sequestering agent of the formula (I) is tris(3,6-dioxaoctyl)-amine of the formula:

$N-(CH_2-CH_2-O-CH_2-CH_2-O-C_2H_5)_3$

8. Process according to Claim 1, characterised in that the complexing compound is a macrocyclic polyether having from 15 to 30 atoms in the ring and consisting of 5 to 10 units -O-X, in which X is either $-CHR_6-CHR_7$ or $-CHR_6-CHR_8-CR_9R_7-$, $R_6$, $R_7$, $R_8$ and $R_9$, which are identical or different, being a hydrogen atom or an alkyl radical having from 1 to 4 carbon atoms, and it being possible for one of the symbols X to be $-CHR_6-CHR_8-CR_9R_7-$ when the units -O-X comprise the group $-O-CHR_6-CHR_7$.

9. Process according to Claim 8, characterised in that the macrocyclic polyether is chosen from amongst the group comprising:

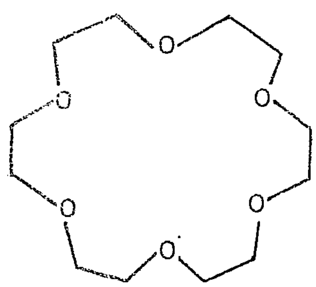

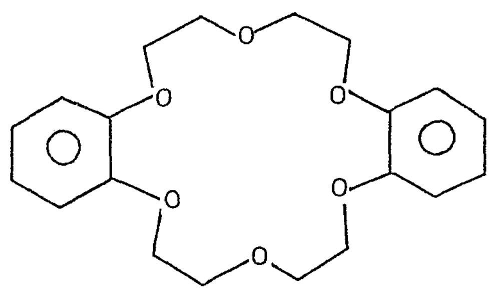

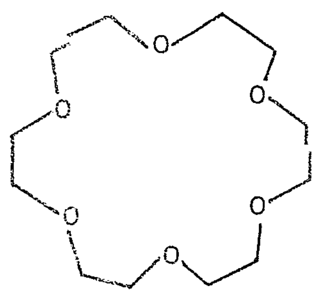

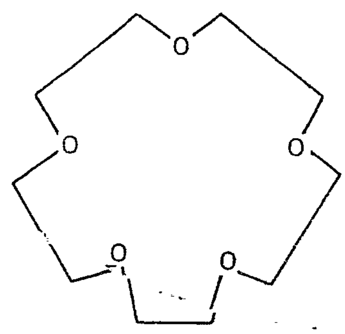

10. Process according to Claim 1, characterised in that the complexing compound is a macrocyclic or bicyclic compound of the general formula IIa or IIb:

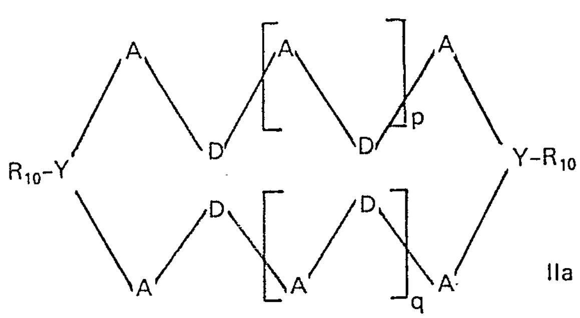

IIa

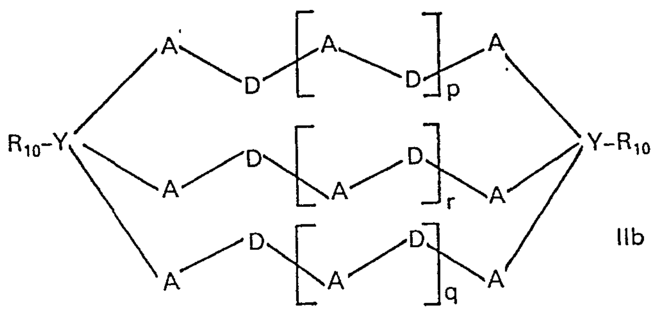

IIb

in which formulae:

- Y represents N or P,
- A represents an alkylene group having from 1 to 3 carbon atoms,
- D represents O, S or N-$R_{11}$, in which $R_{11}$ represents an alkyl radical having from 1 to 6 carbon atoms, and
- $R_{10}$ represents an alkyl radical having from 1 to 6 carbon atoms, p, q and r, which are identical or different, being integers between 1 and 5.

11. Process according to Claim 10, characterised in that the macrocyclic or bicyclic compound is chosen from amongst the group comprising:

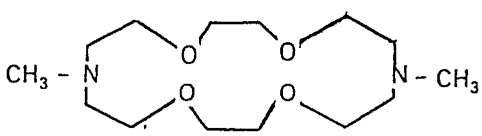

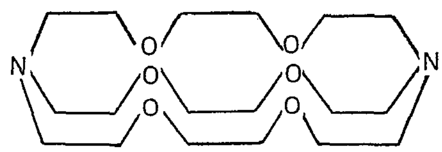

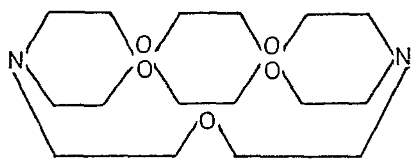

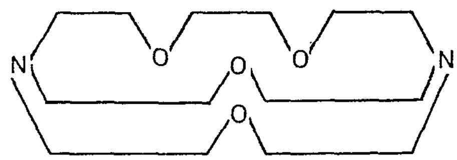

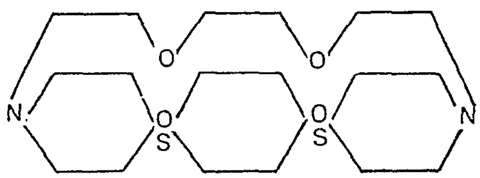

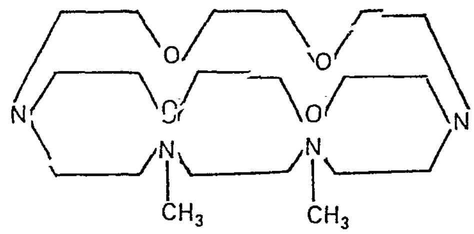

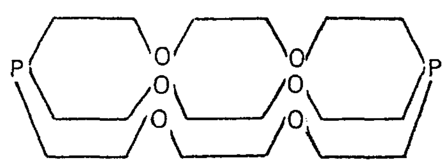

12. Process according to any one of the preceding claims, characterised in that the reaction is carried out in the presence of an aromatic solvent.

13. Process according to Claim 12, characterised in that the solvent is chosen from amongst the group comprising chlorobenzene, benzene and toluene.

14. Process according to any one of the prceding claims, characterised in that the alkali metal amide is lithium, potassium or sodium amide.

15. Process according to any one of the preceding claims, characterised in that the alkali metal alcoholate is chosen from amongst the group comprising the compounds $RO^-M^+$, in which $M^+$ represents sodium or potassium and in which R represents an alkyl radical containing from 1 to 6 carbon atoms.

16. Process according to any one of the preceding claims, characterised in that the reaction is carried out at a temperature of between −20°C and 60°C.

17. Process according to any one of the preceding claims, characterised in that the molar ratio of the alkali metal amide to the sequestering agent of the formula (I) is between 0.005 and 0.5.

18. Process according to any one of the preceding claims, characterised in that the ratio of the alkali metal amide to the starting polychlorobenzene(s) is between 1 and 0.05.

19. Process according to any one of the preceding claims, for the preparation of 1,2,5-trichlorobenzene, characterised in that at least one tetrachlorobenzene, if appropriate mixed with 1,2,3-trichlorobenzene and/or 1,2,4-trichlorobenzene and/or pentachlorobenzene and/or hexachlorobenzene, is reacted.

20. Process according co Claim 19, characterised in that at least one tetrachlorobenzene mixed with 1,2,4-trichlorobenzene is reacted.

21. Process according to Claim 19, characterised in that a mixture of 1,2,4,5-tetrachlorobenzene and 1,2,4-trichlorobenzene is reacted.

22. Process according to either one of Claims 19 and 20, characterised in that the molar ratio of tetrachloro to trichloro is between 0.1 and 10.